(19) Europäisches Patentamt
European Patent Office
Office européen des brevets

(11) Publication number: 0 035 285 A2

(12) **EUROPEAN PATENT APPLICATION**

(21) Application number: 81102642.6

(51) Int. Cl.³: C 07 D 261/18, A 61 K 31/42

(22) Date of filing: 17.08.79

(43) Date of publication of application: 09.09.81 Bulletin 81/36

(84) Designated Contracting States: BE CH DE FR GB IT NL SE

(60) Publication number of the earlier application in accordance with Art. 76 EPC: 0016277

(71) Applicant: AMERICAN CYANAMID COMPANY, Berdan Avenue, Wayne New Jersey 06904 (US)

(72) Inventor: Hanifin, John William, Jr., 2 Sunset Terrace, Suffern New York (US)
Inventor: Ridge, David Nelson, Old Mountain Road, Upper Grandview New York (US)

(74) Representative: Allam, Peter Clerk et al, LLOYD WISE, TREGEAR & CO. Norman House 105-109 Strand, London WC2R 0AE (GB)

(54) **Novel isoxazole carboxylic acid phenyl esters, pharmaceutical compositions containing certain of said esters, and process for preparing said esters.**

(57) There are provided novel isoxazole carboxylic acid phenyl esters of the formula:

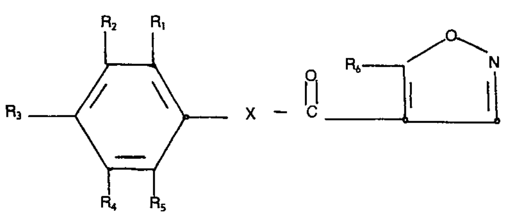

....I

wherein $R_1$, $R_2$, $R_3$, $R_4$ and $R_5$ are each hydrogen, halogen, lower alkyl ($C_1$-$C_4$), lower alkoxy ($C_1$-$C_4$), trifluoromethyl or trichloromethyl; $R_6$ is lower alkyl ($C_1$-$C_4$); and X is sulfur or oxygen.

The novel compounds are useful intermediates for the preparation of certain anti-inflammatory substituted cis-2-benzoyl-3-hydroxy-alkenonitriles forming the subject of European Patent Application No. 0016277A. Certain of the novel compounds themselves exhibit anti-inflammatory activity.

EP 0 035 285 A2

TITLE: NOVEL ISOXAZOLE CARBOXYLIC ACID PHENYL ESTERS, PHARMACEUTICAL COMPOSITIONS CONTAINING CERTAIN OF SAID ESTERS, AND PROCESS FOR PREPARING SAID ESTERS

This invention relates to novel isoxazole carboxylic acid phenyl esters which are useful as intermediates for the preparation of certain anti-inflammatory agents disclosed in our co-pending European Application No. 0016277A (Serial No. 79301687.4) from which the present application is divided.

According to this invention there is provided compounds of the formula:

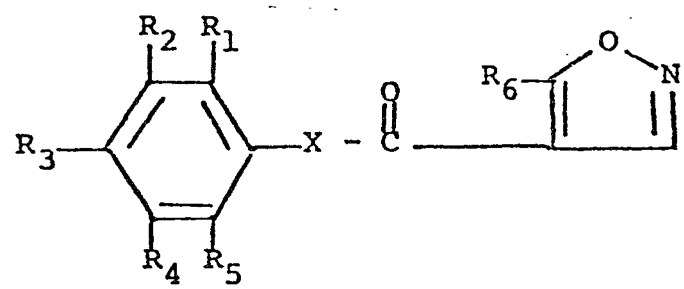

I

wherein $R_1$, $R_2$, $R_3$, $R_4$ and $R_5$ are each selected from hydrogen, halogen, lower alkyl ($C_1$-$C_4$), lower alkoxy ($C_1$-$C_4$), trifluoromethyl and trichloromethyl; $R_6$ is lower alkyl ($C_1$-$C_4$); and X is sulfur or oxygen.

The novel compounds of this invention may be treated with triethylamine in ether solution followed by acidification to produce compounds of the formula:

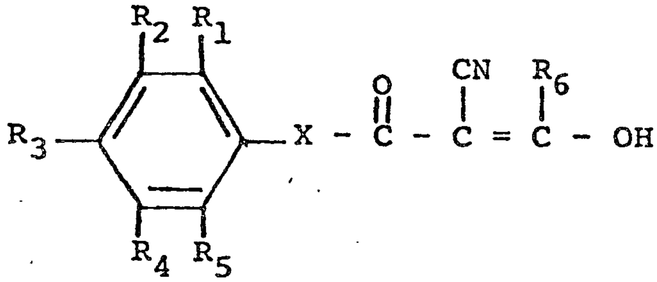

wherein $R_1$, $R_2$, $R_3$, $R_4$, $R_5$, $R_6$ and X are as described above for Formula I, which compounds are active anti-inflammatory agents and form the subject of the parent application, *supra*.

In addition, two of the novel compounds of Formula I, namely 5-methyl-4-isoxazolecarboxylic acid, *o*-methoxyphenyl ester and 5-methyl-4-isoxazolecarboxylic acid, 2,4,6-trichlorophenyl ester, are themselves active as anti-inflammatory agents.

The compounds of Formula I may be prepared according to the following Flowchart A:

FLOWCHART A

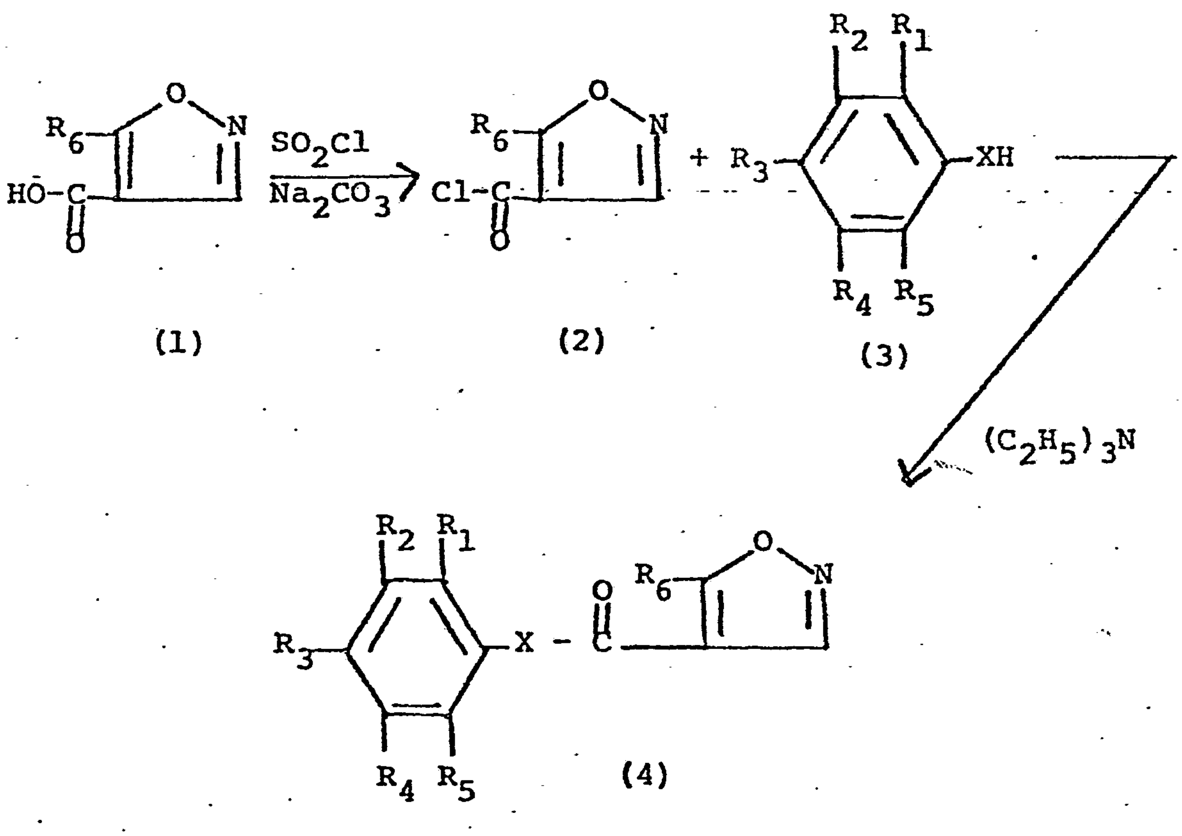


(1) (2) (3) (4)

In accordance with Flowchart A a 5-alkylisoxazole -4-carboxylic acid (1) is converted to the corresponding 5-alkylisoxazole carbonyl chloride (2) by treatment with thionyl chloride and sodium carbonate at reflux for several hours. The chloride (2) is then reacted with the substituted phenol or thiophenol (3) in ether with one molar equivalent of triethylamine added. The reaction mixture is filtered, the filtrate evaporated and the residue extracted from methylene chloride to give the product (4), where $R_1$, $R_2$, $R_3$, $R_4$, $R_5$, $R_6$ and X are as described above for Formula I.

As indicated above, two of the novel compounds of the present invention have been found to be active for meliorating inflammation and inhibiting joint deterioration in mammals. For this purpose, they may be administered in amounts ranging from about one milligram to about 250 mg per kilogram of body weight per day. A preferred dosage regimen for optimum results would be from about 5 mg to about 100 mg per kilogram of body weight per day, and such dosage units are employed that a total of from about 0.35 gm to about 7.0 gm of the active ingredient for a subject of about 79 kg of body weight are administered in a 24 hour period. This dosage regimen may be adjusted to provide the optimum therapeutic response. For example, several divided doses may be administered daily or the dose may be proportionally reduced as indicated by the exigencies of the therapeutic situation. A decided practical advantage of this invention is that the active ingredient may be administered in any convenient manner such as by the oral, intravenous, intramuscular, topical, intraarticular, or subcutaneous route. The anti-inflammatory activity of certain of the novel compounds of the present invention was established by the following test.

Synovium-cartilage Test

Compounds of the invention have been tested by the synovium-cartilage test which is a modification of the method described by Dumonde, D.C. and Glynn, L.E., The Production of Arthritis in Rabbits by an Immunological Reaction to Fibrin, Brit. J. Exp. Pathol., 43, 373 (1962).

Male, New Zealand rabbits, weighing 1.6-3.0 kg were used. The rabbits were housed in individual cages and given food and water *ad libitum*. The rabbits were sensitized to bovine serum albumin (BSA) by injection of 20 mg./kg. BSA in complete Freund's adjuvant. The emulsion was injected subcutaneously into multiple sites on the back. Total injection volume equaled one mg./kg. of body weight. Between 18 and 21 days following immunization, the rabbits were challenged with an intra-articular injection of 5 mg. BSA into the right knee joint. Unanesthetized animals were restrained on their backs, and a 20-gauge, one-inch needle, with a 5 ml. syringe, was introduced into the joint and synovial fluid aspirated to determine that the synovium had been penetrated. The needle was then left in place for injection of the antigen. A 0.5 ml. portion of 10 mg./ml. BSA in sterile saline was injected into the synovial sac. The rabbits received an additional five intra-articular injections of 5 mg. BSA into the right knee at intervals of 12-16 days. Following the sixth injection the rabbits were sacrificed by an intracardiac injection of 5 ml. of a saturated solution of potassium chloride. The right knee was shaved. A longitudinal incision was made over the patellar ligament and the skin reflected under sterile conditions. The patellar ligament was cut transversely and reflected. Fascia and capsular tissue were trimmed from the lateral and medial aspects of the knee exposing the synovial membrane. The membrane was grasped with forceps, stretched laterally and then excised in a single piece on each side of the knee. The infrapatellar fat pad was removed and the joint space widened by cutting the anterior attachments of the menisci and severing the

collateral cruciate ligaments. The femur and tibia were then separated and the menisci excised along with sufficient amounts of synovial tissue from the popliteal area adhering to the menisci. The tissues were immediately placed in a sterile Petri dish containing tissue culture medium composed of MEM (Earle's salts) without sodium bicarbonate, with 25 mM Hepes buffer, pH adjusted to 7.34-7.37, and antibiotics (streptomycin and neomycin, 100 units/ml.). Ten per cent normal rabbit serum (NRS) was added. The tissues were rinsed three times in fresh medium plus 10% NRS, then cut into pieces of 20-30 mg.

Articular cartilage was obtained from the knees of normal, young rabbits, weighing 1.0 to 1.5 kg. The knees were shaved, the rabbits sacrificed and the joint exposed as described above. Synovial tissue and the infrapatellar fat pad were removed. Ligaments were severed and the menisci excised. Femur and tibia were then separated and articular cartilage was cut from supracondylar lines, patellar surface and femoral condyles. Due to curvature of the bone, these pieces were not more than 6-7 mg. each. No cartilage was taken from the tibia. The cartilage was placed in a sterile Petri dish containing tissue culture medium plus 10% NRS and rinsed three times with fresh medium + 10% NRS. The cartilage was then cut into 1-2 mg. pieces and stored at -70°C.

A 10 mg. portion of the test compound was dissolved or suspended in absolute ethanol. Ten μl. were then transferred to the complete tissue culture medium. The final concentration of test compound was then 10 mcg./ml. and the vehicle 0.1%.

Tissue culture medium was added to 12 x 75 mm clear plastic culture tubes containing one piece of normal articular cartilage. A piece of arthritic synovial tissue was then added to all tubes except those tubes in which cartilage was incubated alone. All tubes were then incubated at 37°C. for 48 hours with constant rotation in a roller drum at 0.2 rpm. After 48 hours the cartilage was removed,

hydrolyzed and assayed for hexoseamine and hydroxyproline. Hexoseamine to hydroxyproline ratios were calculated for the three groups:

1. Cartilage alone
2. Cartilage + synovium
3. Cartilage + synovium + test compound.

Cartilage hexoseamine decreases when it is cultured in the presence of synovium but remains constant when cultured alone. Hydroxyproline remains constant in all groups and the amount assayed is a measure of the size of the incubated cartilage. Therefore, the hexoseamine/-hydroxyproline ratio decreases in the cartilage + synovium group relative to the cartilage alone group. The decrease is approximately 50%.

If a compound prevents the decrease in the hexoseamine/hydroxyproline ratio by greater than 50% it is retested. A compound is considered active if it averages greater than 50% suppression of breakdown in three separate tests.

The following Table I gives the results of this test with compounds of the present invention. These compounds are indicated to be active when tested by the above procedure.

TABLE I

| Compound | No. of Tests | Mean % Inhibition |
|---|---|---|
| 5-Methyl-4-isoxazolecarboxylic acid, o-methoxyphenyl ester | 3 | 86.67 |
| 5-Methyl-4-isoxazolecarboxylic acid, 2,4,6-trichlorophenyl ester | 3 | 63.80 |

Pharmaceutical compositions according to the present invention having the desired clarity, stability and adaptability for parenteral and intra-articular use are obtained by dissolving from 0.10% to 10.0% by weight of active compound in a vehicle consisting of a polyhydric aliphatic alcohol or mixtures thereof. Especially satisfactory are glycerin, propylene glycol, and polyethylene glycols. The polyethylene glycols consist of a mixture of non-volatile, normally liquid, polyethylene glycols which are soluble in both water and organic liquids and which have molecular weights of from about 200 to 1500. Although the amount of active compound dissolved in the above vehicle may vary from 0.10% to 10.0% by weight, it is preferred that the amount of active compound employed be from about 3.0% to about 9.0% by weight. Although various mixtures of the aforementioned non-volatile polyethylene glycols may be employed, it is preferred to use a mixture having an average molecular weight of from about 200 to about 400.

In addition to the active compound, the parenteral solutions may also contain various preservatives which may be used to prevent bacterial and fungal contamination. The preservatives which may be used for these purposes are, for example, myristyl-gamma-picolinium chloride, phenyl mercuric nitrate, benzalkonium chloride, phenethyl alcohol, p-chlorophenyl-α-glycerol ether, methyl and propyl parabens, and thimerosal. As a practical matter it is also convenient to employ antioxidants. Suitable antioxidants include, for example, sodium bisulfite, sodium metabisulfite, and sodium formaldehyde sulfoxylate. Generally, from about 0.05 to about 0.2% concentrations of antioxidant are employed.

For intramuscular injection, the preferred concentration of active compound is 0.25 to 0.50 mg./ml. of the finished compositions. The active compounds of the invention are equally adapted to intravenous administration when diluted with water or diluents employed in intravenous therapy such as isotonic glucose in appropriate quantities. For intravenous use, initial concentrations down to about

0.05 to 0.25 mg./ml. of active compound are satisfactory.

The active compounds of the present invention may be orally administered, for example, with an inert diluent or with an assimilable edible carrier, or they may be enclosed in hard or soft shell gelatin capsules, or they may be compressed into tablets, or they may be incorporated directly with the food of the diet. For oral therapeutic administration, the active compounds may be incorporated with excipients and used in the form of tablets, troches, capsules, elixirs, suspensions, syrups, wafers, and the like. Such compositions and preparations should contain at least 0.1% of active compound. The percentage of the compositions and preparations may, of course, be varied and may conveniently be between about 2% to about 60% of the weight of the unit. The amount of active ingredient in such therapeutically useful compositions is such that a suitable dosage will be obtained. Preferred compositions or preparations according to the present invention are prepared so that an oral dosage unit form contains between about 50 and 250 milligrams of active compound.

The tablets, troches, pills, capsules and the like may also contain the following: a binder such as gum tragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, lactose or saccharin may be added or a flavoring agent such as peppermint, oil of wintergreen, or cherry flavoring. When the dosage unit form is a capsule, it may contain, in addition to materials of the above type, a liquid carrier such as a fatty oil. Various other materials may be present as coatings or to otherwise modify the physical form of the dosage unit. For instance, tablets, pills, or capsules may be coated with shellac, sugar or both. A syrup or elixir may contain the active compound, sucrose as a sweetening agent, methyl and propylparabens as preservatives, a dye and flavoring such as cherry or orange flavor. Of course, any

material used in preparing any dosage unit form should be pharmaceutically pure and substantially non-toxic in the amounts employed.

The invention is illustrated by the following Examples.

## EXAMPLE 1

## 5-Methylthio-4-Isoxazole Carboxylic Acid-S-Phenyl Ester

A 72 ml. portion of thionyl chloride is added dropwise to a mixture of 70.24 g. of 5-methylisoxazole-4-carboxylic acid [H. Yasuda, Yakugaku Zasshi, 79, 836-838 (1959); C.A. 53, 21885d] and 64.44 g. of sodium carbonate in 250 ml. of chloroform. The mixture is heated gently on a steam bath for 4 hours, then the solid is filtered and the filtrate is evaporated to an oil. This oil is distilled at 4.5 mm. and the material boiling at 68-70°C. is collected, giving 65.23 g. of 5-methylisoxazole-4-carbonyl chloride.

A mixture of 10.27 ml. (11.02 g.) of thiophenol and 11.15 ml. (14.6 g.) of 5-methylisoxazole-4-carbonyl chloride in 50 ml. of ether is cooled in an ice bath. A 14 ml. portion of triethylamine is diluted to 50 ml. with ether and added dropwise. The mixture is stirred for 2 hours and the solid is filtered and washed with ether. The filtrate is evaporated to an oil which crystallizes, is taken up in methylene chloride and filtered through diatomaceous earth. The filtrate is concentrated on a steam bath with the addition of hexanes, given an oil. Cooling crystallizes this oil given 16.65 g of the desired product, 5-methylthio-4-isoxazolecarboxylic acid-S-phenyl ester, as a whith crystalline solid. This product may be used to prepare 2-cyano-3-hydroxythiocrotonic acid-S-phenyl ester, as described in Example 120 of the parent Application 0016277A.

## EXAMPLE 2

### 5-Methylthio-4-Isoxazolecarboxylic acid-S-(p-chlorophenyl) ester

A 14.46 g. portion of p-chlorothiophenol and 11.15 ml. (14.6 g.) of 5-methylisoxazole-4-carbonyl chloride in 50 ml. of ether is cooled in an ice bath and a solution of 14 ml. of triethylamine in 50 ml. of ether is added dropwise. The mixture is stirred for 3 hours and then diluted with ether. The solid is filtered and washed with ether. The combined filtrate and washing is evaporated to a solid which is taken up in methylene chloride, filtered and evaporated on a steam bath with the addition of hexanes giving an oil which crystallizes on cooling, giving the desired product, 5-methylthio-4-isoxazolecarboxylic acid-S-(p-chlorophenyl) ester, as a white crystalline solid. This product may be used to prepare 2-cyano-3-hydroxylthiocrotonic acid-S-(p-chlorophenyl) ester as described in Example 121 of the parent Application 0016277A.

Following the general procedure of Examples 1 and 2, other representative compounds of this invention, such as those found in Table II, may be made. These compounds may be converted into anti-inflammatory 2-carbonyl-3-hydroxy-alkenonitriles, as shown in Examples 122-132 of the parent Application 0016277A.

TABLE II

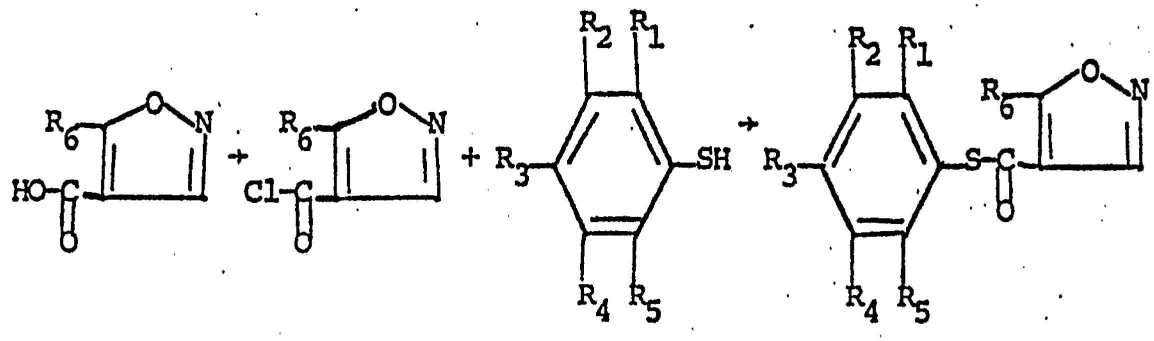

(1) (2) (3) (4)

TABLE II (continued)

| Example | Alkyl Isoxazole Acid (1) | Alkyl Isoxazole Chloride (2) | Thio-phenol (3) | Alkylthio Isoxazole Phenyl Ester (4) | Method of |
|---|---|---|---|---|---|
| 3 | $R_6=CH_3$ | $R_6=CH_3$ | $R_1$, $R_2$, $R_4$, $R_5=H$ $R_3=CH_3$ | $R_1$, $R_2$, $R_4$, $R_5=H$ $R_3=CH_3$ $R_6=CH_3$ | Example 1 |
| 4 | $R_6=CH_3$ | $R_6=CH_3$ | $R_1$, $R_2$ $R_4$, $R_5=H$ $R_3=F$ | $R_1$, $R_2$ $R_4$, $R_5=H$ $R_3=F$ $R_6=CH_3$ | Example 2 |
| 5 | $R_6=CH_3$ | $R_6=CH_3$ | $R_1-R_3$, $R_5=H$ $R_4=CH_3$ | $R_1-R_3$, $R_5=H$ $R_4$, $R_6=CH_3$ | Example 1 |
| 6 | $R_6=CH_3$ | $R_6=CH_3$ | $R_1-R_3$, $R_5=H$ $R_4=Cl$ | $R_1-R_3$, $R_5=H$ $R_4=Cl$ $R_6=CH_3$ | Example 2 |

TABLE II (continued)

| Example | Alkyl Isoxazole Acid (1) | Alkyl Isoxazole Chloride (2) | Thio-phenol (3) | Alkylthio Isoxazole Phenyl Ester (4) | Method of |
|---|---|---|---|---|---|
| 7 | $R_6=CH_3$ | $R_6=CH_3$ | $R_2-R_5=H$ $R_1=Cl$ | $R_2-R_5=H$ $R_1=Cl$ $R_6=CH_3$ | Example 2 |
| 8 | $R_6=CH_3$ | $R_6=CH_3$ | $R_2-R_5=H$ $R_1=CH_3O$ | $R_2-R_5=H$ $R_1=CH_3O$ $R_6=CH_3$ | Example 2 |
| 9 | $R_6=CH_3$ | $R_6=CH_3$ | $R_1-R_3$, $R_5=H$ | $R_1-R_3$, $R_5=H$ $R_4=CH_3O$ $R_6=CH_3$ | Example 2 |
| 10 | $R_6=CH_3$ | $R_6=CH_3$ | $R_2-R_5=H$ $R_1$, $R_6=CH_3$ | $R_2-R_5=H$ $R_1$, $R_6=CH_3$ | Example 1 |

TABLE II (continued)

| Example | Alkyl Isoxazole Acid (1) | Alkyl Isoxazole Chloride (2) | Thio-phenol (3) | Alkylthio Isoxazole Phenyl Ester (4) | Method of |
|---|---|---|---|---|---|
| 11 | $R_6=CH_3$ | $R_6=CH_3$ | $R_1$, $R_2$, $R_4$, $R_5=H$ $R_3=CF_3$ $R_6=CH_3$ | $R_1$, $R_2$, $R_4$, $R_5=H$ $R_3=CF_3$ $R_6=CH_3$ | Example 1 |
| 12 | $R_6=CH_3$ | $R_6=CH_3$ | $R_1-R_3$, $R_5=H$ $R_4=CF_3$ $R_6=CH_3$ | $R_1-R_3$, $R_5=H$ $R_4=CF_3$ $R_6=CH_3$ | Example 1 |
| 13 | $R_6=CH_3$ | $R_6=CH_3$ | $R_1-R_3$, $R_5=H$ $R_4Br$ $R_6=CH_3$ | $R_1-R_3$, $R_5=H$ $R_4Br$ $R_6=CH_3$ | Example 1 |

EXAMPLE 14

5-Methyl-4-Isoxazolecarboxylic acid, p-chlorophenyl ester

A 130 ml. portion of thionyl chloride is added slowly to a mixture of 63.67 g. of 5-methylisoxazole-4-carboxylic acid [H. Yasuda, Yakugaku Zasshi, 79, 836-838 (1959); C.A., 53, 21885d] and 58.4 g. of sodium carbonate in 250 ml. of chloroform. The mixture is heated on a steam bath for 2 hours, filtered and the solvents are removed from the filtrate by evaporation. Two portions of chloroform are successively added to the residue and removed by evaporation. The resulting oil is distilled at 1-2 mm and the material boiling at 66-68°C. is collected, giving 64.48 g. of 5-methylisoxazole carbonyl chloride.

A mixture of 12.86 g. of p-chlorophenol and 11.5 ml. (14.6 g.) of 5-methylisoxazole carbonyl chloride in 100 ml. of ether is cooled in an ice bath. A 14 ml. portion of triethylamine in 50 ml. of ether is added dropwise. The mixture is stirred for one hour and then filtered. The filtrate is evaporated to an oil which is distilled. The fraction boiling at 132-137°C is collected, giving 14.85 g of the desired product, 5-methyl-4-isoxazolecarboxylic acid, p-chlorophenyl ester. This product may be used to prepare 2-cyano-3-hydroxycrotonic acid, p-chlorophenyl ester, as described in Example 133 of the parent Application 0016277A.

EXAMPLE 15

5-Methyl-4-Isoxazolecarboxylic acid, 2,3-dichlorophenyl ester

A 16.3 g portion of 2,3-dichlorophenol and 11.15 ml (14.6 g) of 5-methylisoxazole carbonyl chloride in 50 ml of ether is cooled in an ice bath and a solution of 14 ml of triethylamine is added dropwise. The mixture is stirred for

3 hours and the solid is filtered and washed with ether. The combined filtrate and wash is evaporated to an oil on a steam bath. This oil is taken up in methylene chloride, filtered through diatonaceous earth and the filtrate evaporated on a steam bath with the addition of hexanes to about 50 ml. Cooling gives 24.02 g of the desired product, 5-methyl-4-isoxazolecarboxylic acid, 2,3-dichlorophenyl ester, as a tan crystalline solid. This product may be used to prepare 2-cyano-3-hydroxycrotonic acid, 2,3-dichlorophenyl ester, as described in Example 134 of the parent Application 0016277A.

Following the general procedure of Examples 14 and 15 other representative compounds of this invention, such as those found in Table III, may be made.

TABLE III

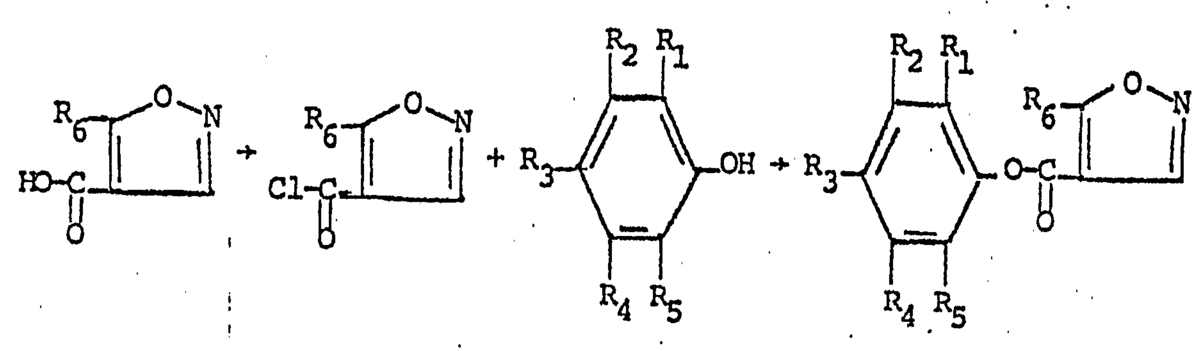

(1) (2) (3) (4)

| Example | Alkylisoxazole Acid (1) $R_6$= | Alkylisoxazole Chloride (2) $R_6$= | Phenol (3) | Alkylisoxazole carboxylic Phenyl Ester (4) | Method of |
|---|---|---|---|---|---|
| 16 | $CH_3$ | $CH_3$ | $R_1$-$R_4$=H<br>$R_5$=Cl | $R_1$-$R_4$=H<br>$R_5$=Cl | Example 14 |
| 17 | $CH_3$ | $CH_3$ | $R_1$, $R_2$, $R_4$, $R_5$= H<br>$R_3$=$CH_3O$ | $R_1$, $R_2$, $R_4$, $R_5$= H<br>$R_3$=$CH_3O$ | Example 15 |
| 18 | $CH_3$ | $CH_3$ | $R_1$, $R_2$, $R_4$, $R_5$=H<br>$R_3$=$CH_3$ | $R_1$, $R_2$, $R_4$, $R_5$=H<br>$R_3$=$CH_3$ | Example 14 |

TABLE III (continued)

| Example | Alkylisoxazole Acid (1) $R_6$= | Alkylisoxazole Chloride (2) $R_6$= | Phenol (3) | Alkylisoxazole carboxylic Phenyl Ester (4) | Method of |
|---|---|---|---|---|---|
| 19 | $CH_3$ | $CH_3$ | $R_1=CH_3O$<br>$R_2-R_5=H$ | $R_1=CH_3O$<br>$R_2-R_5=H$ | Example 15 |
| 20 | $CH_3$ | $CH_3$ | $R_1-R_4=H$<br>$R_5=F$ | $R_1-R_4=H$<br>$R_5=F$ | Example 14 |
| 21 | $CH_3$ | $CH_3$ | $R_2$, $R_3$, $R_4$, $R_5$= H<br>$R_1=CH_3O$ | $R_2$, $R_3$, $R_4$, $R_5$=H<br>$R_1=CH_3O$ | Example 15 |
| 22 | $CH_3$ | $CH_3$ | $R_1$, $R_2$, $R_5$=H<br>$R_3$, $R_4$=Cl | $R_1$, $R_2$, $R_5$=H<br>$R_3$, $R_4$=Cl | Example 15 |
| 23 | $CH_3$ | $CH_3$ | $R_1$, $R_2$, $R_4$, $R_5$=H<br>$R_3$=F | $R_1$, $R_2$, $R_4$, $R_5$=H<br>$R_3$=F | Example 14 |

TABLE III (continued)

| Example | Alkylisoxazole Acid (1) $R_6$= | Alkylisoxazole Chloride (2) $R_6$= | Phenol (3) | Alkylisoxazole carboxylic Phenyl Ester (4) | Method of |
|---|---|---|---|---|---|
| 24 | $CH_3$ | $CH_3$ | $R_1$, $R_4$, $R_5$=H $R_2$, $R_3$= $CH_3O$ | $R_1$, $R_4$, $R_5$=H $R_2$, $R_3$= $CH_3O$ | Example 15 |
| 25 | $CH_3$ | $CH_3$ | $R_1$, $R_2$, $R_4$, $R_5$=H $R_3$=Br | $R_1$, $R_2$, $R_4$, $R_5$=H $R_3$=Br | Example 14 |
| 26 | $CH_3$ | $CH_3$ | $R_2$, $R_3$, $R_4$, $R_5$=H $R_1$=Br | $R_2$, $R_3$, $R_4$, $R_5$=H $R_1$=Br | Example 14 |
| 27 | $CH_3$ | $CH_3$ | $R_1$, $R_3$, $R_4$, $R_5$=H $R_2$=Br | $R_1$, $R_3$, $R_4$, $R_5$=H $R_2$=Br | Example 14 |
| 28 | $CH_3$ | $CH_3$ | $R_1$, $R_2$, $R_3$, $R_4$, $R_5$=H | $R_1$, $R_2$, $R_3$, $R_4$, $R_5$=H | Example 14 |

EXAMPLE 29

5-Methyl-4-isoxazolecarboxylic acid, o-methoxyphenyl ester

A 186 ml portion of thionylchloride is added dropwise to a solution of 181.67 g of 5-methylisoxazole-4-carboxylic acid (H. Yasuda, Yakugaku Zasshi, 79, 836-838 (1959); C.A., 53 21885d) and 167 g of sodium carbonate in 500 ml of chloroform. The reaction is refluxed gently on a steam bath for 4 hours and then stirred at room temperature overnight. The mixture is filtered and the filtrate is evaporated to an oil which is distilled at 3.5 mm. The product, 5-methylisoxazole carbonyl chloride is recovered from the fraction boiling at 61°C (3.5 mm), yield 183.43 g.

A mixture of 145.5 g of 5-methylisoxazole carbonyl chloride and 124.1 g of o-methoxyphenol in 50 ml of ether is cooled in an ice bath. A 14 ml portion of triethylamine in 50 ml of ether is added dropwise. The mixture is stirred for 2 hours, filtered and washed with ether. The combined filtrate and wash is evaporated. The residue is taken up in methylene chloride, filtered through diatomaceous earth and evaporated on a steam bath, with the addition of hexanes, to an oil. Cooling the oil gives the desired product as 4.47 g of a white crystalline solid, m.p. 108-111°C.

Following the general procedure of Example 29, other representative compounds of this invention, such as those found in Table IV, may be made.

TABLE IV

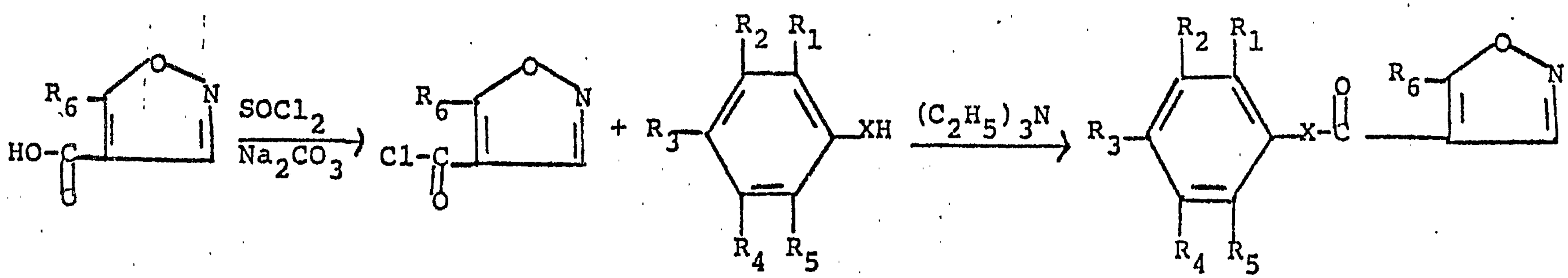


(1) (2) (3) (4)

Table IV (cont'd)

| Example | Isoxazole Carboxylic Acid (1) | Isoxazole Carbonyl Chloride (2) | Phenyl Derivative (3) | Isoxazole Phenyl Ester (4) | M.P. °C. |
|---|---|---|---|---|---|
| 30 | $R_6$=$CH_3$ | $R_6$=$CH_3$ | $R_2$, $R_4$=H<br>$R_1$, $R_3$<br>$R_5$=Cl<br>X=O | $R_2$, $R_4$=H/<br>$R_1$, $R_3$<br>$R_5$=Cl<br>X=O<br>$R_6$=$CH_3$ | 85-87 |
| 31 | $R_6$=$CH_3$ | $R_6$=$CH_3$ | $R_1$, $R_2$,<br>$R_4$, $R_5$=H<br>$R_3$=F<br>X=O | $R_1$, $R_2$,<br>$R_4$, $R_5$=H<br>$R_3$=F<br>X=O<br>$R_6$=$CH_3$ | Oil |
| 32 | $R_6$=$CH_3$ | $R_6$=$CH_3$ | $R_1$, $R_2$,<br>$R_4$, $R_5$=H<br>$R_3$=$CH_3O$<br>X=O | $R_1$, $R_2$<br>$R_4$, $R_5$=H<br>$R_3$=$CH_3O$<br>X=O<br>$R_6$=$CH_3$ | 62-69 |
| 33 | $R_6$=$CH_3$ | $R_6$=$CH_3$ | $R_1$, $R_2$,<br>$R_3$, $R_5$=H<br>$R_4$=$CF_3$<br>X=O | $R_1$, $R_2$,<br>$R_3$, $R_5$=H<br>$R_4$=$CF_3$<br>X=O<br>$R_6$=$CH_3$ | Oil |
| 34 | $R_6$=$CH_3$ | $R_6$=$CH_3$ | $R_1$, $R_2$,<br>$R_4$, $R_5$=H<br>$R_3$=Cl<br>X=O | $R_1$, $R_2$<br>$R_4$, $R_5$=H<br>$R_3$=Cl<br>X=O<br>$R_6$=$CH_3$ | Oil |
| 35 | $R_6$=$CH_3$ | $R_6$=$CH_3$ | $R_1$-$R_4$=H<br>$R_5$=$CF_3$<br>X=O | $R_1$-$R_4$=H<br>$R_5$=$CF_3$<br>X=O<br>$R_6$=$CH_3$ | Oil |

Table IV (cont'd)

| Example | Isoxazole Carboxylic Acid (1) | Isoxazole Carbonyl Chloride (2) | Phenyl Derivative (3) | Isoxazole Phenyl Ester (4) | M.P. °C. |
|---|---|---|---|---|---|
| 36 | $R_6$=$CH_3$ | $R_6$=$CH_3$ | $R_1$, $R_3$, $R_4$, $R_5$=H<br>$R_2$=$CH_3O$<br>X=O | $R_1$, $R_3$, $R_4$, $R_5$=H<br>$R_2$=$CH_3O$<br>X=O<br>$R_6$=$CH_3$ | Oil |
| 37 | $R_6$=$CH_3$ | $R_6$=$CH_3$ | $R_1$-$R_4$=H<br>$R_5$=Cl<br>X=O | $R_1$-$R_4$=H<br>$R_5$=Cl<br>X=O<br>$R_6$=$CH_3$ | 71-76 |
| 38 | $R_6$=$CH_3$ | $R_6$=$CH_3$ | $R_1$, $R_2$, $R_4$, $R_5$=H<br>$R_3$=$CH_3$<br>X=O | $R_1$, $R_2$, $R_4$, $R_5$=H<br>$R_3$, $R_6$=$CH_3$<br>X=O | Oil |
| 39 | $R_6$=$CH_3$ | $R_6$=$CH_3$ | $R_1$-$R_4$=H<br>$R_5$=$CH_3$<br>X=O | $R_1$-$R_4$=H<br>$R_5$, $R_6$=$CH_3$<br>X=O | Oil |
| 40 | $R_6$=$CH_3$ | $R_6$=$CH_3$ | $R_1$, $R_2$, $R_3$, $R_5$=H<br>$R_4$=Cl<br>X=O | $R_1$, $R_2$, $R_3$, $R_5$=H<br>$R_4$=Cl<br>X=O<br>$R_6$=$CH_3$ | 38-39 |
| 41 | $R_6$=$CH_3$ | $R_6$=$CH_3$ | $R_1$, $R_2$, $R_5$=H<br>$R_3$=Cl<br>$R_4$=$CH_3$<br>X=O | $R_1$, $R_2$, $R_5$=H<br>$R_3$=Cl<br>$R_4$, $R_6$=$CH_3$<br>X=O | Oil |
| 42 | $R_6$=$CH_3$ | $R_6$=$CH_3$ | $R_1$, $R_2$, $R_4$=H<br>$R_3$, $R_5$=Cl<br>X=O | $R_1$, $R_2$, $R_4$=H<br>$R_3$, $R_5$=Cl<br>X=O<br>$R_6$=$CH_3$ | 91-92 |

Table IV (cont'd)

| Example | Isoxazole Carboxylic Acid (1) | Isoxazole Carbonyl Chloride (2) | Phenyl Derivative (3) | Isoxazole Phenyl Ester (4) | M.P. °C. |
|---|---|---|---|---|---|
| 43 | $R_6=CH_3$ | $R_6=CH_3$ | $R_2-R_4=H$ $R_1$, $R_5=Cl$ X=O | $R_2-R_4=H$ $R_1$, $R_5=Cl$ X=O $R_6=CH_3$ | 96-101 |
| 44 | $R_6=CH_3$ | $R_6=CH_3$ | $R_1$, $R_2$, $R_5=H$ $R_3$, $R_4=Cl$ X=O | $R_1$, $R_2$ $R_5=H$ $R_3$, $R_4=Cl$ X=O $R_6=CH_3$ | 86-93 |
| 45 | $R_6=CH_3$ | $R_6=CH_3$ | $R_1-R_3=H$ $R_4$, $R_5=Cl$ X=O | $R_1-R_3=H$ $R_4$, $R_5=Cl$ X=O $R_6=CH_3$ | 88-91 |
| 46 | $R_6=CH_3$ | $R_6=CH_3$ | $R_1$, $R_2$, $R_3$, $R_5=H$ $R_4=CH_3$ X=O | $R_1$, $R_2$ $R_3$, $R_5=H$ $R_4$, $R_6=CH_3$ X=O | Oil |
| 47 | $R_6=CH_3$ | $R_6=CH_3$ | $R_1-R_4=H$ $R_5=F$ X=O | $R_1-R_4=H$ $R_5=F$ X=O $R_6=CH_3$ | 58-65 |
| 48 | $R_6=CH_3$ | $R_6=CH_3$ | $R_1$, $R_3$, $R_5=H$ $R_2$, $R_4=CF_3$ X=O | $R_1$, $R_3$, $R_5=H$ $R_2$, $R_4=CF_3$ X=O $R_6=CH_3$ | 76-78 |
| 48 | $R_6=CH_3$ | $R_6=CH_3$ | $R_1$, $R_4$, $R_5=H$, $R_2$, $R_3=CH_3O$ X=O | $R_1$, $R_4$, $R_5=H$, $R_2$, $R_3=CH_3O$ X=O, $R_6=CH_3$ | 75-79 |

Table IV (cont'd)

| Example | Isoxazole Carboxylic Acid (1) | Isoxazole Carbonyl Chloride (2) | Phenyl Derivative (3) | Isoxazole Phenyl Ester (4) | M.P. °C. |
|---|---|---|---|---|---|
| 50 | $R_6=CH_3$ | $R_6=CH_3$ | $R_1$, $R_2$, $R_4$, $R_5=H$ $R_3=Br$ $X=O$ | $R_1$, $R_2$, $R_4$, $R_5=H$ $R_3=Br$ $X=O$ $R_6=CH_3$ | Oil |
| 51 | $R_6=CH_3$ | $R_6=CH_3$ | $R_2-R_5=H$ $R_1=Br$ $X=O$ | $R_2-R_5=H$ $R_1=Br$ $X=O$ $R_6=CH_3$ | 65-68 |
| 52 | $R_6=CH_3$ | $R_6=CH_3$ | $R_1$, $R_2$, $R_3$, $R_5=H$ $R_4=Br$ $X=O$ | $R_1$, $R_2$, $R_3$, $R_5=H$ $R_4=Br$ $X=O$ $R_6=CH_3$ | Oil |
| 53 | $R_6=CH_3$ | $R_6=CH_3$ | $R_1-R_5=H$ $X=S$ | $R_1-R_5=H$ $X=S$ $R_6=CH_3$ | |
| 54 | $R_6=CH_3$ | $R_6=CH_3$ | $R_1$, $R_2$, $R_4$, $R_5=H$ $R_3=Cl$ $X=S$ | $R_1$, $R_2$, $R_4$, $R_5=H$ $R_3=Cl$ $X=S$ $R_6=CH_3$ | 90-92 |
| 55 | $R_6=CH_3$ | $R_6=CH_3$ | $R_1$, $R_2$, $R_4$, $R_5=H$ $R_3=CH_3$ $X=S$ | $R_1$, $R_2$, $R_4$, $R_5=H$ $R_3$, $R_6=CH_3$ $X=S$ | 41-44 |
| 56 | $R_6=CH_3$ | $R_6=CH_3$ | $R_1$, $R_2$, $R_4$, $R_5=H$ $R_3=F$, $X=S$ | $R_1$, $R_2$, $R_4$, $R_5=H$, $R_3=F$ $X=S$, $R_6=CH_3$ | 66-70 |

Table IV (cont'd)

| Example | Isoxazole Carboxylic Acid (1) | Isoxazole Carbonyl Chloride (2) | Phenyl Derivative (3) | Isoxazole Phenyl Ester (4) | M.P. °C. |
|---|---|---|---|---|---|
| 57 | $R_6=CH_3$ | $R_6=CH_3$ | $R_1$, $R_2$, $R_3$, $R_5=H$ $R_4=CH_3$ X=S | $R_1$, $R_2$, $R_3$, $R_5=H$ $R_4$, $R_6=CH_3$ X=S | Oil |
| 58 | $R_6=CH_3$ | $R_6=CH_3$ | $R_1$, $R_2$, $R_3$, $R_5=H$ $R_4=Cl$ X=S | $R_1$, $R_2$, $R_3$, $R_5=H$ $R_4=Cl$ X=S, $R_6=CH_3$ | Oil |
| 59 | $R_6=CH_3$ | $R_6=CH_3$ | $R_1-R_4=H$ $R_5=Cl$ X=S | $R_1-R_4=H$ $R_5=Cl$ X=S $R_6=CH_3$ | 83-87 |
| 60 | $R_6=CH_3$ | $R_6=CH_3$ | $R_1-R_4=H$ $R_5=CH_3O$ X=S | $R_1-R_4=H$ $R_5=CH_3O$ X=S $R_6=CH_3$ | 69-72 |
| 61 | $R_6=CH_3$ | $R_6=CH_3$ | $R_1$, $R_3$, $R_4$, $R_5=H$ $R_2=CH_3O$ X=S | $R_1$, $R_3$, $R_4$, $R_5=H$ $R_2=CH_3O$ X=S $R_6=CH_3$ | Oil |
| 62 | $R_6=CH_3$ | $R_6=CH_3$ | $R_2-R_5=H$ $R_1=CH_3$ X=S | $R_2-R_5=H$ $R_1$, $R_6=CH_3$ X=S | 61-64 |

CLAIMS:

1. A compound of the formula:

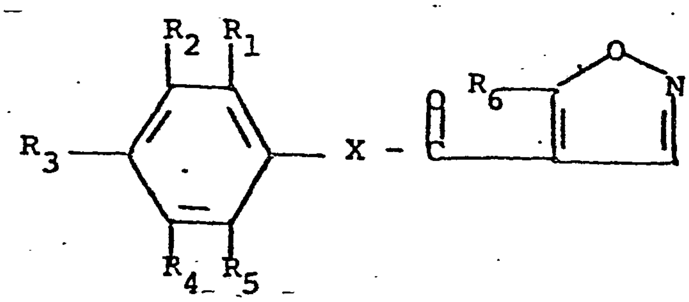

I

wherein $R_1$, $R_2$, $R_3$, $R_4$ and $R_5$ are each hydrogen, halogen, lower alkyl ($C_1$-$C_4$), lower alkoxy ($C_1$-$C_4$), trifluoromethyl or trichloromethyl; $R_6$ is lower alkyl ($C_1$-$C_4$); and X is sulfur or oxygen.

2. A compound according to Claim 1, wherein $R_6$ is methyl, X is O and three of the radicals $R_1$, $R_2$, $R_3$, $R_4$ and $R_5$ are chloro, the remaining of said radicals being hydrogen; or two of said radicals are both chloro, both methoxy or both trifluoromethyl, the remaining being hydrogen; or one of said radicals is chloro, another is methyl and the remaining are hydrogen; or one of said radicals is bromo, chloro, fluoro, methyl, methoxy or trifluoromethyl and the remaining are hydrogen.

3. A compound according to Claim 1, wherein $R_6$ is methyl, X is S and one of the radicals $R_1$, $R_2$, $R_3$, $R_4$ and $R_5$ is chloro, fluoro, methyl or methoxy, and the remaining of said radicals are hydrogen; or all of said radicals are hydrogen.

4. An anti-arthritic composition useful in inhibiting the progression of arthritis, meliorating inflammation and/or inhibiting progressive joint deterioration in mammals, comprising a pharmaceutically acceptable carrier or diluent and a compound selected from 5-methyl-4-isoxazolecarboxylic acid, o-methoxyphenyl ester and 5-methyl-4-isoxazolecarboxylic acid, 2,4,6-trichlorophenyl ester.

5. A method of producing compounds of Formula I as claimed in Claim 1, which comprises reacting a compound of the formula:

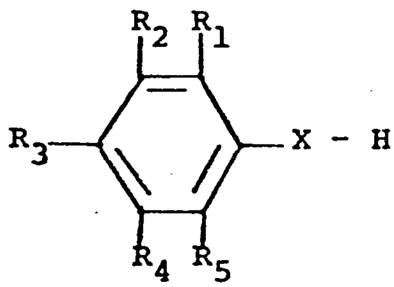

with a compound of the formula:

halide -

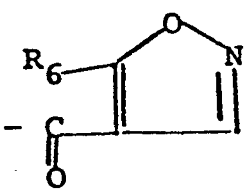

in the presence of a proton scavenger such as a carbonate, bicarbonate or tertiary amine, wherein X and $R_1$, $R_2$, $R_3$, $R_4$ and $R_5$ are as defined for Formula I.

LLOYD WISE, TREGEAR & CO.
NORMAN HOUSE 105-109 STRAND
LONDON, WC2R 0AE